(19) Europäisches Patentamt
European Patent Office
Office européen des brevets

(11) Veröffentlichungsnummer: **0 195 974**
**B1**

(12) # EUROPÄISCHE PATENTSCHRIFT

(45) Veröffentlichungstag der Patentschrift: **29.11.89**

(51) Int. Cl.⁴: **C07C 161/04**, C07C 121/60, C07D 239/26, C09K 19/08

(21) Anmeldenummer: **86103200.1**

(22) Anmeldetag: **10.03.86**

(54) **Alkenylsubstituierte Phenylisothiocyanate und Benzonitrile.**

(30) Priorität: **26.03.85 CH 1316/85**
**08.01.86 CH 32/86**

(43) Veröffentlichungstag der Anmeldung:
**01.10.86 Patentblatt 86/40**

(45) Bekanntmachung des Hinweises auf die Patenterteilung:
**29.11.89 Patentblatt 89/48**

(84) Benannte Vertragsstaaten:
**CH DE FR GB IT LI SE**

(56) Entgegenhaltungen:
**EP-A- 0 084 194**
**EP-A- 0 126 883**
**EP-A- 0 169 327**
**AT-B- 356 120**
**CH-A- 646 725**
**DE-A- 2 636 684**
**GB-A- 2 121 406**

(73) Patentinhaber: **F. HOFFMANN-LA ROCHE AG, Postfach 3255, CH-4002 Basel(CH)**

(72) Erfinder: **Buchecker, Richard, Dr., Dornacherstrasse 146, CH-4053 Basel(CH)**
Erfinder: **Schadt, Martin, Dr., Liestalerstrasse 77, CH-4411 Seltisberg(CH)**

(74) Vertreter: **Zimmermann, Hans, Dr. et al, Grenzacherstrasse 124 Postfach 3255, CH-4002 Basel(CH)**

EP 0 195 974 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue alkenylsubstituierte Phenylisothiocyanate und Benzonitrile sowie flüssigkristalline Gemische, welche diese Verbindungen enthalten, und die Verwendung der Verbindungen und Mischungen für elektro-optische Zwecke.

Flüssige Kristalle haben vor allem als Dielektrika in Anzeigevorrichtungen Bedeutung erlangt, da die optischen Eigenschaften solcher Substanzen durch eine elektrische Spannung beeinflusst werden können. Elektro-optische Vorrichtungen auf der Basis von Flüssigkristallen sind dem Fachmann bekannt und können auf verschiedenen Effekten beruhen, wie beispielsweise der dynamischen Streuung, der Deformation aufgerichteter Phasen (DAP-Typ), dem Schadt-Helfrich-Effekt (Drehzelle), dem Gast/Wirt-Effekt (Guest/Host-Zelle) oder einem cholesterisch-nematischen Phasenübergang (Phase-Change-Zelle).

Damit Flüssigkristalle als Dielektrika für electro-optische Anzeigevorrichtungen geeignet sind, müssen sie jedoch einer Reihe von Anforderungen genügen. Beispielsweise müssen sie eine gute chemische Stabilität gegenüber Umwelteinflüssen, wie z. B. Wärme, Feuchtigkeit, Luft und electromagnetische Strahlung in infraroten, sichtbaren und ultravioletten Bereich besitzen. Ferner sollten sie farblos sein, einen guten Kontrast ergeben und im ganzen Temperaturbereich, in welchem die Flüssigkristallzelle betrieben werden soll, eine nematische bzw. cholesterische Mesophase besitzen. Weiterhin sollten die Flüssigkristalle niedere Viskositäten und kurze Ansprechzeiten aufweisen. Letzteres ist besonders bei Anwendung in Fernsehgeräten wichtig. Für Anzeigen mit hoher Informationsdichte sind ferner eine hohe Multiplexierbarkeit und somit steile Transmissionskennlinien erforderlich. Weitere Eigenschaften müssen je nach verwendetem Zellentyp unterschiedliche Bedingungen erfüllen. Beispielsweise sollten Flüssigkristalle, die in Drehzellen verwendet werden, eine grosse positive Anisotropie der Dielektrizitätskonstante ($\Delta\varepsilon = \varepsilon_{\parallel} - \varepsilon_{\perp} > O$ wobei $\varepsilon_{\parallel}$ die Dielektrizitätskonstante entlang der Moleküllängsachse und $\varepsilon_{\perp}$ die Dielektrizitätskonstante senkrecht dazu bedeuten), und Flüssigkristalle, die in Guest/Host-Zellen verwendet werden, eine grosse positive oder negative Anisotropie der Dielektrizitätskonstante aufweisen. In beiden Fällen ist zudem eine niedrige Schwellenspannung und eine möglichst geringe Leitfähigkeit erwünscht.

Da es im allgemeinen nicht möglich ist, alle gewünschten und zum Teil widersprüchlichen Eigenschaften mit einer einzigen Verbindung zu erreichen, wird meist versucht, durch Mischen mehrerer Komponenten die Eigenschaften für die jeweiligen Anwendungen zu optimieren. Im Hinblick auf die hohen Anforderungen bei Anwendungen mit hoher Informations-dichte und insbesondere bei Anwendungen in Fernsehgeräten und Autos besteht jedoch ein Bedarf an weiteren Komponenten, mit denen sich insbesondere die Ansprechzeiten und die Steilheit der Transmissionskennlinie weiter verbessern lassen.

Es wurde nun gefunden, dass die alkenylsubstituierten Verbindungen der allgemeinen Formel

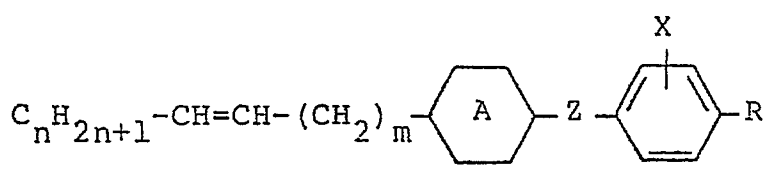

IA

und insbesondere die alkenylsubstituierten Phenylisothiocyanate der allgemeinen Formel

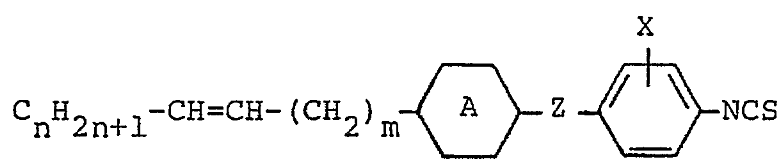

I

worin m für eine ganze Zahl von 4-7 und n für null oder eine positive ganze Zahl steht; Ring A trans-1,4-Cyclohexylen oder einen 2,5-disubstituierten Pyrimidinring darstellt; Z eine einfache Kovalenzbindung oder, sofern Ring A trans-1,4-Cyclohexylen darstellt, auch die Aethylengruppe $-CH_2CH_2-$ bedeutet; X Wasserstoff oder Fluor bezeichnet; und R die Gruppe -NCS oder -CN darstellt,
die gewünschten Eigenschaften aufweisen.

Die erfindungsgemässen Phenylisothiocyanate besitzen insbesondere ein günstiges Verhältnis der elastischen Konstanten $k_{33}$ (bend) und $k_{11}$ (splay) und ergeben bei Anwendung in elektro-optischen Vorrichtungen steile Transmissionskurven und eine hohe Multiplexierbarkeit. Zudem weisen sie eine grosse positive Anisotropie der Dielektrizitätskonstante auf und ermöglichen niedere Betriebsspannungen. Weiterhin besitzen die erfindungsgemässen Verbindungen niedere Viskositäten und ergeben bei Anwendungen in elektro-optischen Vorrichtungen sehr Kurze Schaltzeiten. Die erfindungsgemässen Verbindungen sind farblos, chemisch stabil und gut mischbar mit bekannten Flüssigkristallen. Die Mesophasenbereiche liegen in einem für elektro-optische Anwendungen günstigen Temperaturbereich, insbesondere liegen die Schmelzpunkte im allgemeinen bei relativ tiefen Temperaturen. Die erfindungsgemässen Benzonitrile (R=CN) sind flüssigkristalline Zwischenprodukte bei der Herstellung der Phenylisothiocyanate und besitzen selbst bereits vergleichbare Eigenschaften wie die Phenylisothiocyanate.

Die obige Formel IA umfasst Isothiocyanate der folgenden allgemeinen Formeln

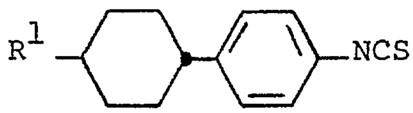

Ia

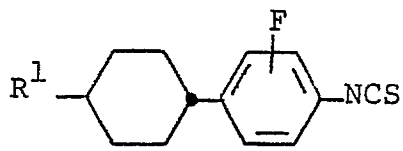

Ib

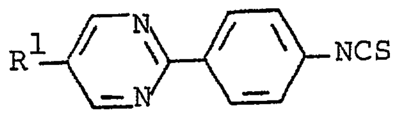

Ic

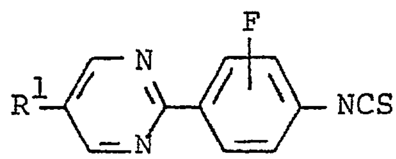

Id

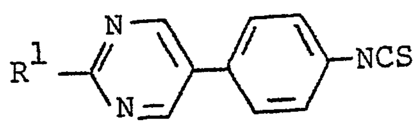

Ie

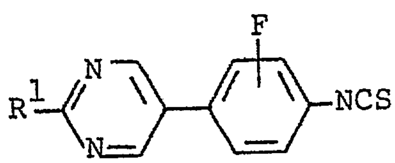

If

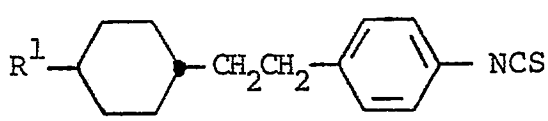

Ig

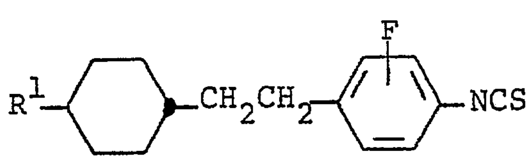

Ih

worin $R^1$ für die Gruppe $C_nH_{2n+1}$-CH=CH-$(CH_2)_m$-
steht und m und n die obigen Bedeutungen haben,
sowie die den Formeln Ia-Ih entsprechenden Benzonitrile, welche anstelle der Gruppe -NCS die Gruppe -CN aufweisen.

In den obigen Formeln IA, I und Ia-Ih bedeutet m die Zahlen 4, 5, 6 und 7 n die Zahlen 0, 1, 2, 3, 4, 5, 6 etc., d.h. die Gruppe $C_nH_{2n+1}$-CH=CH-$(CH_2)_m$- bzw. $R^1$ steht für 5-Alkenyl, wie 5-Hexenyl, 5-Heptenyl, 5-Octenyl, 5-Nonenyl, 5-Decenyl, 5-Undecenyl und 5-Dodecenyl, für 6-Alkenyl, wie 6-Heptenyl, 6-Octenyl, 6-Nonenyl, 6-Decenyl, 6-Undecenyl und 6-Dodecenyl, für 7-Alkenyl, wie 7-Octenyl, 7-Nonenyl, 7-Decenyl, 7-Undecenyl und 7-Dodecenyl, oder für 8-Alkenyl, wie 8-Nonenyl, 8-Decenyl, 8-Undecenyl und 8-Dodecenyl. Die Alkenylgruppen können für n>0 in E- oder Z-Form vorliegen, d.h. die Formeln IA, I und Ia-Ih umfassen sowohl reine Isomere als auch Isomerengemische.

Ein gegebenenfalls vorhandener, lateraler Fluorsubstituent X steht vorzugsweise in ortho-Stellung zur Gruppe -NCS bzw. -CN. Besonders bevorzugt sind jedoch Verbindungen, worin X Wasserstoff bedeutet, d.h. die Verbindungen der Formeln Ia, Ic, Ie und Ig und die entsprechenden Benzonitrile.

In obigen Formeln IA und I bedeutet Z vorzugsweise eine einfache Kovalenzbindung. Ring A bezeichnet vorzugsweise trans-1,4-Cyclohexylen.

Der Rest $C_nH_{2n+1}$ umfasst neben Wasserstoff (für n = 0) sowohl geradkettige als auch verzweigte Alkylgruppen. Bevorzugt sind Wasserstoff und geradkettiges Alkyl, d.h. Reste der Formel H-$(CH_2)_n$-.

Bevorzugte Alkenylsubstituenten in obigen Formeln IA, I und Ia-Ih sind diejenigen mit höchstens 12 Kohlenstoffatomen, d.h. die Summe m+n beträgt vorzugsweise höchstens 10.

Die Verbindungen der Formel I können erfindungsgemäss dadurch hergestellt werden, dass man eine Verbindung der allgemeinen Formel

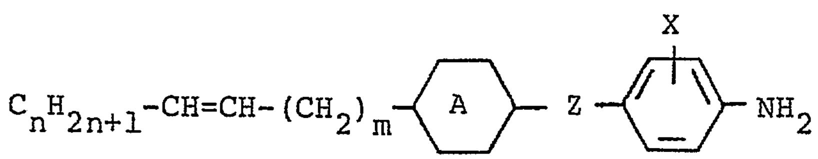

II

worin m, n, X, Z und Ring A die obigen Bedeutungen haben,
in Gegenwart eines Amins mit Thiophosgen oder nacheinander mit Schwefelkohlenstoff und Chlorameisensäureester umsetzt.

Die Umsetzung der Verbindungen der Formel II kann in an sich bekannter Weise erfolgen. Bevorzugte Amine sind Dialkylamine und Trialkylamine, insbesondere diejenigen, worin "Alkyl" jeweils für Gruppen mit 1 bis 7 Kohlenstoffatomen steht. Besonders bevorzugt ist Triäthylamin. Bevorzugte Chlorameisensäureester sind die Chlorameisensäurealkylester, worin der Alkylteil 1 bis 7 Kohlenstoffatome aufweist, wie Chlorameisensäuremethylester und Chlorameisensäureäthylester. Die Reaktion wird zweckmässig in einem polaren organischen Lösungsmittel durchgeführt, beispielsweise einem Aether, chlorierten Kohlenwasserstoff oder Nitril, wie Tetrahydrofuran, Dioxan, Methylenchlorid, Chloroform oder Acetonitril. Das bei der Umsetzung mit Schwefelkohlenstoff erhaltene Ammonium-dithiocarbamat kann gewünschtenfalls isoliert oder direkt mit Chlorameisensäureester weiter umgesetzt werden. Bevorzugt ist jedoch die Umsetzung mit Thiophosgen, welche direkt zum gewünschten Isothiocyanat führt. Temperatur und Druck in dem erfindungsgemässen Verfahren sind nicht kritisch. Vorzugsweise wird jedoch bei Atmosphärendruck und einer Temperatur von etwa 0-40°C gearbeitet.

Die Verbindungen der Formel II sind neu. Sie können beispielsweise aus den entsprechenden Benzonitrilen nach folgendem Reaktionsschema 1 hergestellt werden, worin m, n, X, Z und Ring A die obigen Bedeutungen haben:

Schema 1

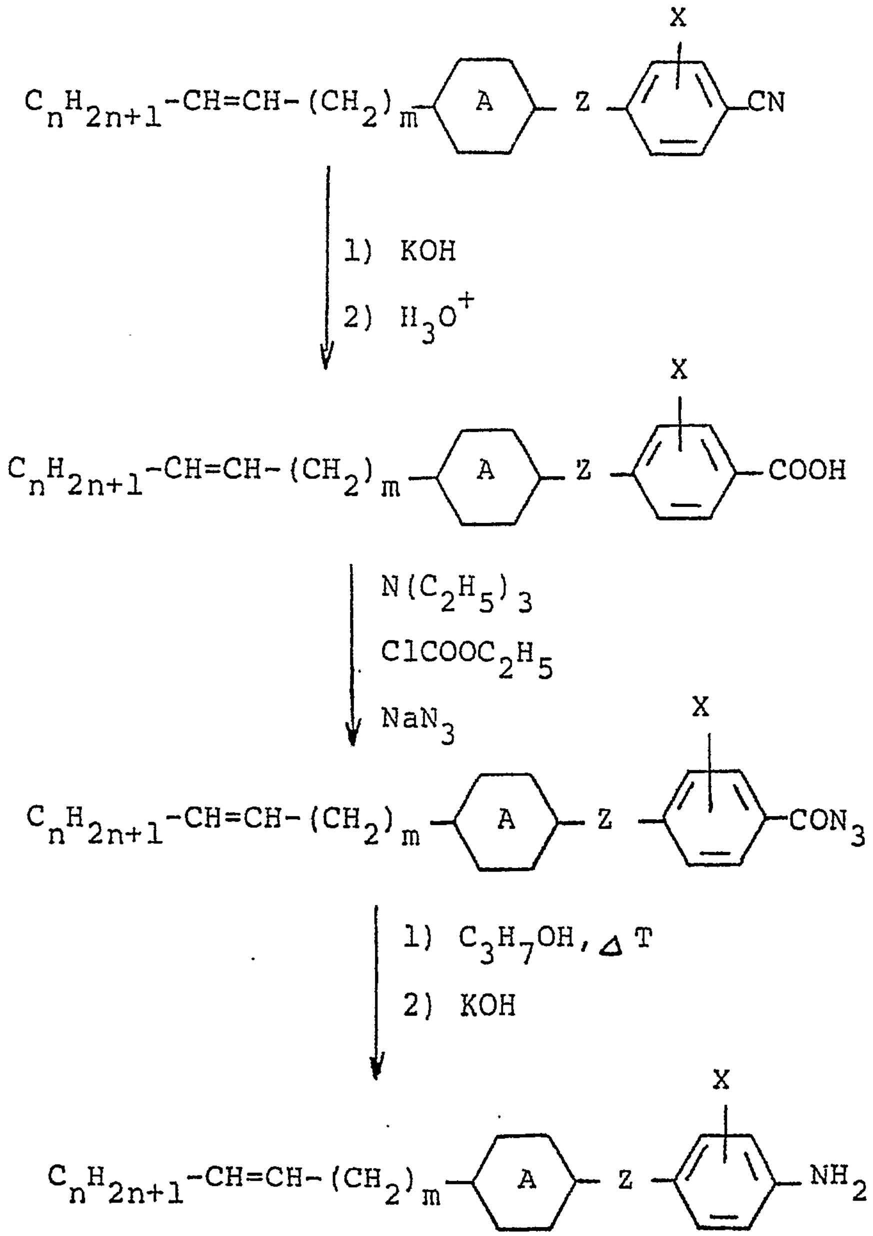


VII

VIII

IX

II

Die Verbindungen der Formel VII sind neu und ebenfalls Gegenstand der vorliegenden Erfindung. Sie besitzen ebenfalls flüssigkristalline Eigenschaften, günstige elastische Konstanten, eine grosse positive Anisotropie der Dielektrizitätskonstante und niedere Viskosität und ermöglichen bei Anwendung in elektro-optischen Vorrichtungen niedere Schwellenspannungen, steile Transmissionkurven und kurze Ansprechzeiten. Sie können in Form von Gemischen untereinander und/oder mit anderen Flüssigkristallkomponenten verwendet werden, insbesondere im Gemisch mit den Verbindungen der Formel I und/oder den weiter unten genannten Verbindungen der Formeln XX-XXIX.

Die Verbindungen der Formel VII können beispielsweise anhand der folgenden Reaktionsschemata 2-4 hergestellt werden, worin Ts p-Tosyl bezeichnet, Z für eine einfache Kovalenzbindung oder die Aethylengruppe $-CH_2CH_2-$ steht und m, n und X die obigen Bedeutungen haben:

Schema 2

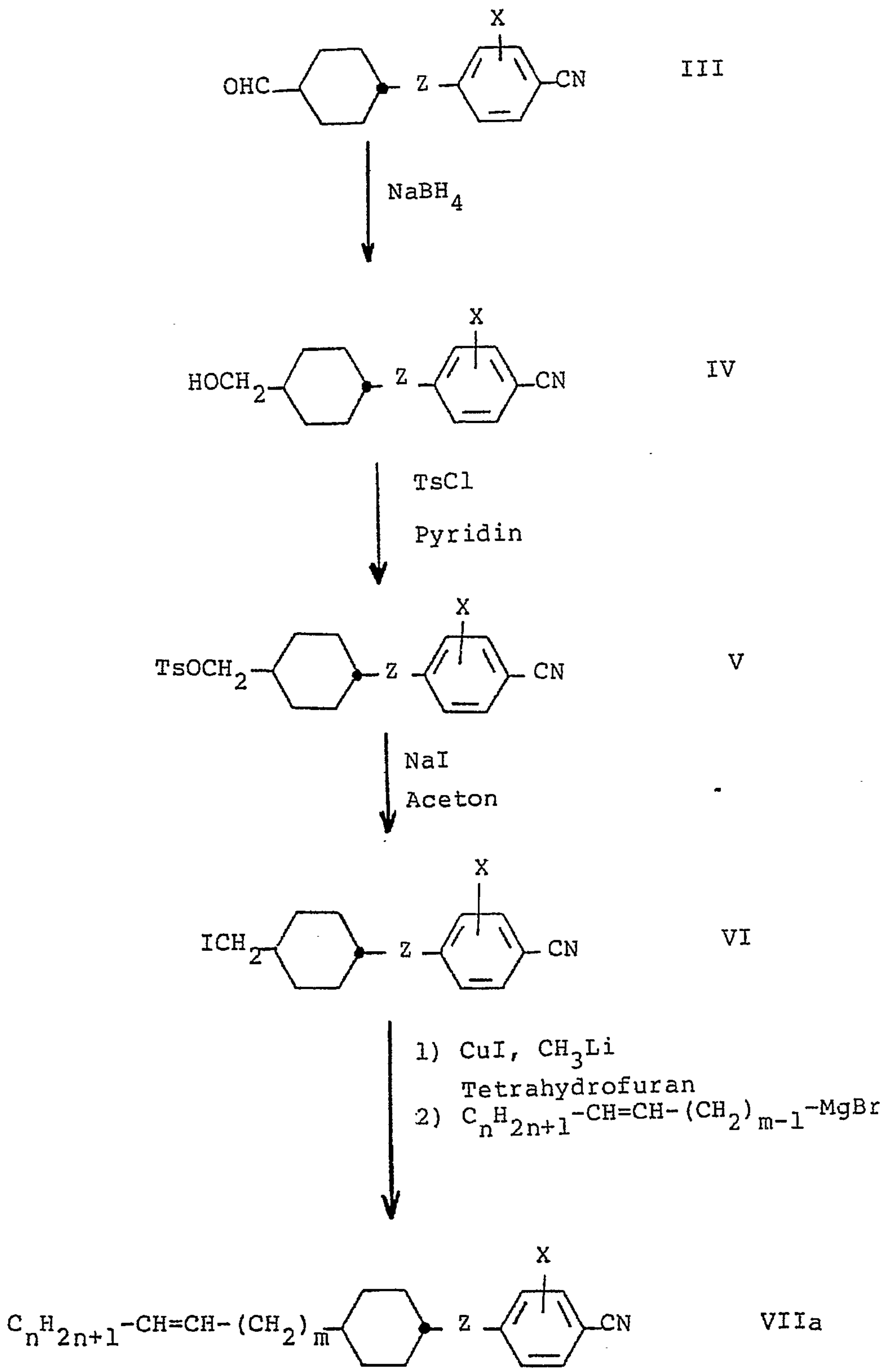


III

IV

V

VI

VIIa

Schema 3

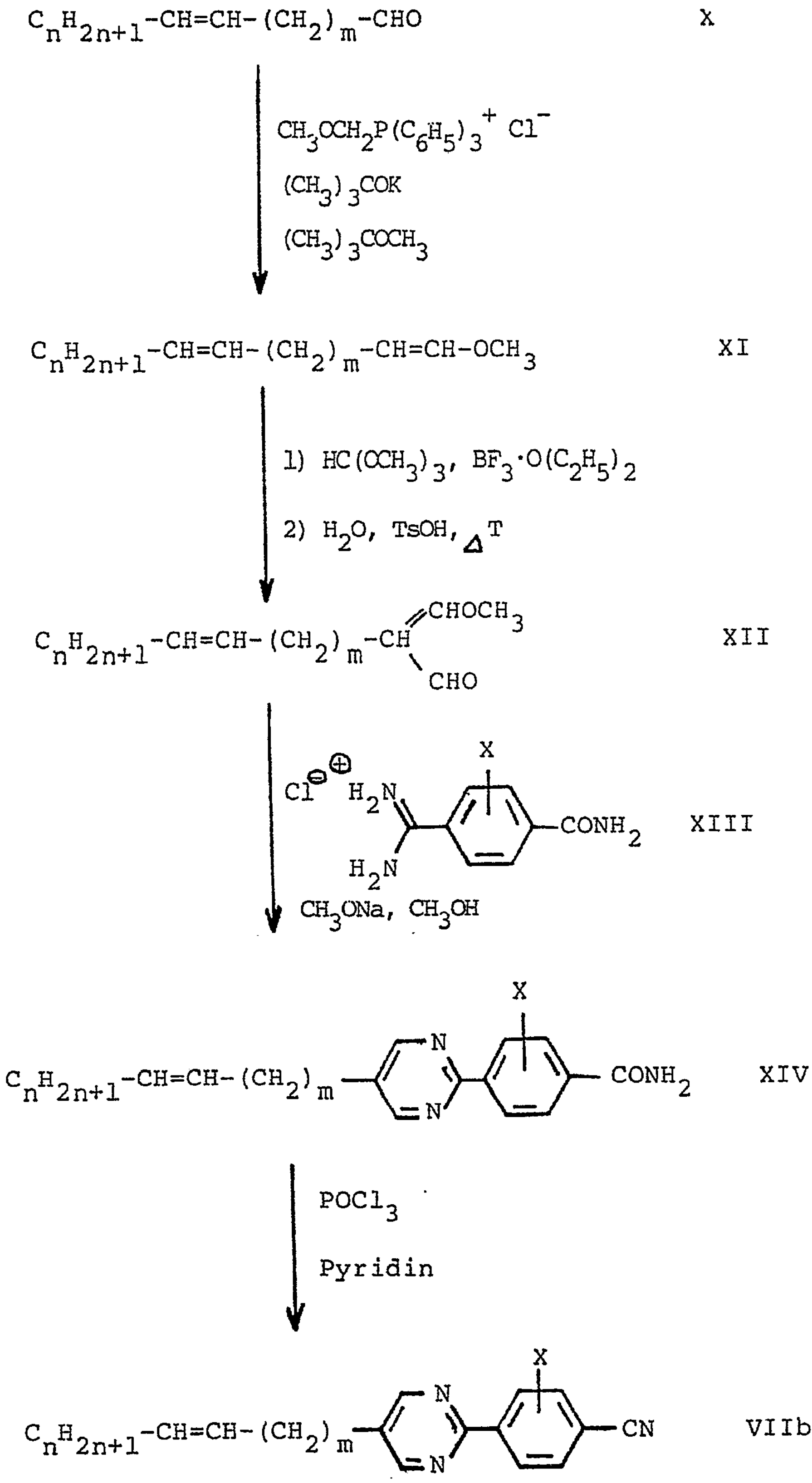


X, XI, XII, XIII, XIV, VIIb

Schema 4

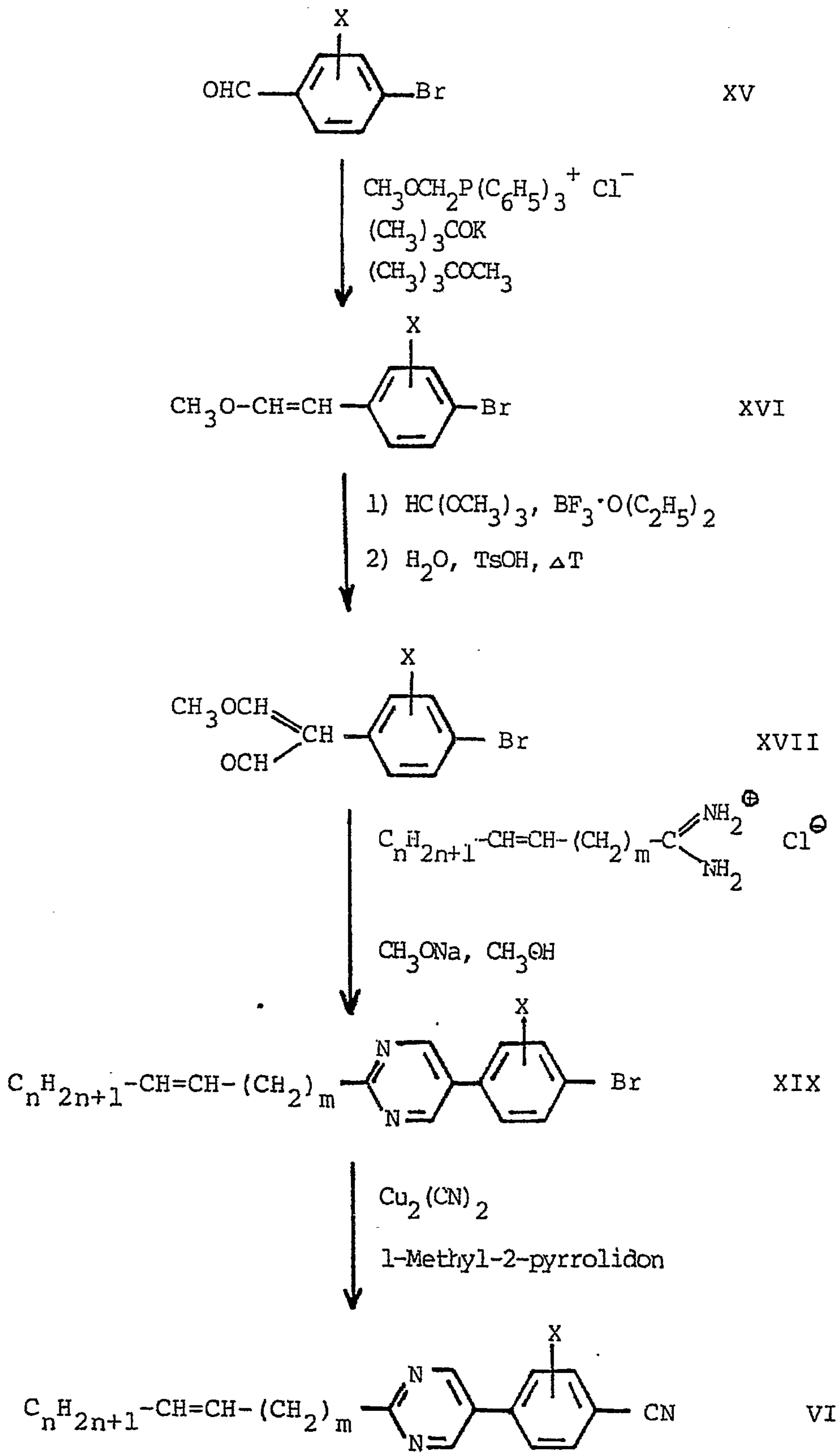
XV
$CH_3OCH_2P(C_6H_5)_3^+ Cl^-$
$(CH_3)_3COK$
$(CH_3)_3COCH_3$
XVI
1) $HC(OCH_3)_3$, $BF_3 \cdot O(C_2H_5)_2$
2) $H_2O$, TsOH, $\Delta T$
XVII
$C_nH_{2n+1}$-CH=CH-$(CH_2)_m$-C(=$NH_2^{\oplus}$)$NH_2$ $Cl^{\ominus}$
XVIII
$CH_3ONa$, $CH_3OH$
XIX
$Cu_2(CN)_2$
1-Methyl-2-pyrrolidon
VIIc

Die in obigen Schemata 2-4 verwendeten Ausgangsmaterialien sind bekannte oder Analoge bekannter Verbindungen und können nach bekannten Methoden hergestellt werden. Die Verbindungen der Formeln XIII und XVIII können beispielsweise aus den entsprechenden Nitrilen durch Umsetzung mit Salzsäure in Methanol und anschliessende Behandlung mit Ammoniak erhalten werden.

Die Verbindungen der Formal IA können in Form von Gemischen untereinander und/oder mit üblichen Flüssigkristallkomponenten verwendet werden, d.h. die erfindungsgemässen Gemische enthalten mindestens zwei Komponenten, wobei mindestens eine Komponente eine Verbindung der Formel IA, vorzugsweise eine Verbindung der Formel I ist.

Aufgrund der günstigen Eigenschaften der Verbindungen der Formel IA und ihrer guten Mischbarkeit und anderen Flüssigkristallkomponenten kann der Anteil der Verbindungen der Formel IA in den erfindungsgemässen Mischungen in breiten Grenzen variieren. Vorzugsweise enthalten die erfindungsgemässen Mischungen etwa 1-60 Gew.-% und besonders bevorzugt etwa 5-40 Gew.-% an Verbindungen der Formel IA bzw. I.

Bevorzugte Komponenten, welche im Gemisch mit einer oder mehreren Verbindungen der Formel IA bzw. I verwendet werden können, sind die Verbindungen der allgemeinen Formel

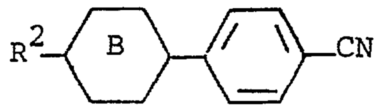

XX

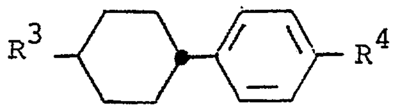

XXI

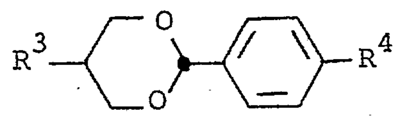

XXII

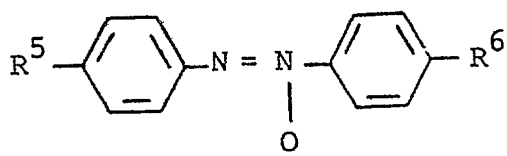

XXIII

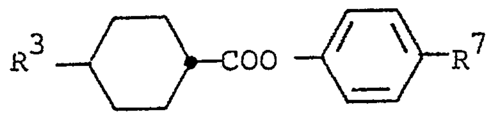

XXIV

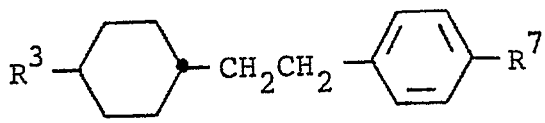

XXV

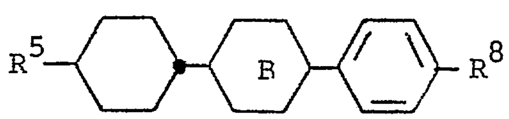

XXVI

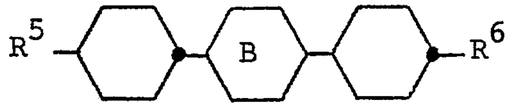

XXVII

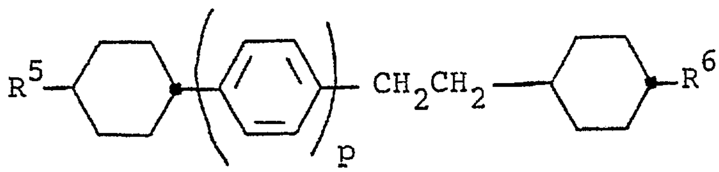

XXVIII

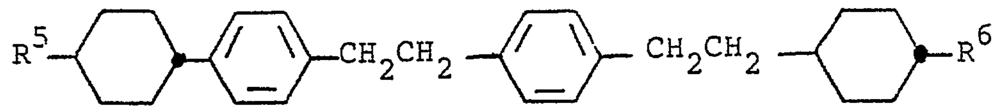

XXIX

worin Ring B 1,4-Phenylen oder einen 2,5-disubstituierten Pyrimidinring darstellt; $R^2$ geradkettiges Alkyl oder 3E-Alkenyl bedeutet; $R^3$ geradkettiges Alkyl, 1E-Alkenyl oder 3E-Alkenyl bezeichnet; $R^4$ für Cyano, -NCS oder geradkettiges Alkyl oder Alkoxy steht; $R^5$ und $R^6$ geradkettiges Alkyl bedeuten; $R^7$ Cyano oder geradkettiges Alkoxy bezeichnet; $R^8$ Cyano oder geradkettiges Alkyl bedeutet; p für 0 oder 1 steht; und die Reste $R^2$-$R^8$ höchstens je 12, vorzugsweise höchstens je 7 Kohlenstoffatome aufweisen.

Die erfindungsgemässen Mischungen können ferner geeignete, optisch aktive Verbindungen, beispielsweise optisch aktive Biphenyle, und/oder dichroitische Farbstoffe, beispielsweise Azo-, Azoxy- oder Anthrachinon-Farbstoffe, enthalten. Der Anteil solcher gerbindungen wird durch die Löslichkeit und die gewünschte Ganghöhe (pitch), Farbe, Extinktion und dergleichen bestimmt. Vorzugsweise beträgt der Anteil optisch aktiver Verbindungen höchstens etwa 4 Gew.-% und der Anteil dichroitischer Farbstoffe höchstens etwa 10 Gew.-%.

Die Herstellung der erfindungsgemässen flüssigkristallinen Gemische kann in an sich bekannter Weise erfolgen, z.B. durch Erhitzen einer Mischung der Bestandteile auf eine Temperatur knapp oberhalb des Klärpunktes und anschliessendes Abkühlen.

Die Herstellung elektro-optischer Vorrichtungen, welche als Dielektrikum eine erfindungsgemässe Mischung enthalten, kann ebenfalls in an sich bekannter Weise erfolgen, z.B. durch Evakuieren einer geeigneten Zelle und Einbringen der Mischung in die evakuierte Zelle.

Die Herstellung der Verbindungen der Formel IA wird durch die folgenden Beispiele weiter veranschaulicht. C bedeutet eine kristalline, S eine smektische, N eine nematische und I die isotrope Phase.

Beispiel 1

a) 60 g trans-4-(p-Cyanophenyl)cyclohexancarboxaldehyd wurden in 500 ml 0,1N methanolischer Kaliumhydroxid-Lösung gelöst und die Lösung innert 1 Stunde bei 0°C portionenweise mit 3,1 g Natriumborhydrid versetzt. Danach wurde das Reaktionsgemisch mit ca. 200 ml Wasser verdünnt und im Eisbad vorsichtig mit ca. 40 ml 25-prozentiger Salzsäure auf pH 2-3 gestellt. Das Gemisch wurde mit Methylenchlorid extrahiert und die organische Phase über Magnesiumsulfat getrocknet und eingeengt. Kristallisation aus einem Gemisch von 250 ml Aethylacetat und 250 ml Petroläther ergab 45,75 g trans-4-(p-Cyanophenyl)-hydroxymethylcyclohexan (Reinheit 99,6%) als farblose Kristalle mit Smp. 108°C.

b) Eine Lösung von 15 g trans-4-(p-Cyanophenyl)hydroxymethylcyclohexan in 60 ml Pyridin wurde bei 0°C mit 22 g p-Tosylchlorid versetzt und dann 19 Stunden bei Raumtemperatur gerührt. Danach wurde das Reaktionsgemisch auf Eiswasser gegossen, mit 25-prozentiger Salzsäure auf pH 1 gestellt und mit Methylenchlorid extrahiert. Die organischen Phasen wurden einmal mit gesättigter Natriumhydrogencarbonat-Lösung und zweimal mit Wasser gewaschen und dann über Magnesiumsulfat getrocknet. Nach Abdampfen des Lösungsmittels wurden 25,58 g rohes trans-4-(p-Cyanophenyl)-p-tosyloxymethylcyclohexan als weisser, kristalliner Rückstand erhalten, welcher ohne weitere Reinigung für die nächste Stufe eingesetzt wurde.

c) 13,4 g rohes trans-4-(p-Cyanophenyl)-p-tosyloxymethylcyclohexan wurden in 200 ml Aceton gelöst und die Lösung mit 8,15 g Natriumjodid versetzt. Das Gemisch wurde 30 Minuten am Rückfluss gekocht, dann eingeengt und zwischen Wasser und Diäthyläther verteilt. Nach Trocknen der organischen Phase über Magnesiumsulfat und Abdampfen des Lösungsmittels wurden 12,5 g gelbliche Kristalle erhalten. Chromatographische Trennung an Kieselgel mit Aethylacetat/Petroläther (Vol. 1:4) und anschliessende Kristallisation aus 140 ml Methanol ergab 3,7 g trans-4-(p-Cyanophenyl)-jodomethylcyclohexan als farblose Kristalle mit Smp. 127°C.

d) Ein Gemisch von 705 mg trockenem Kupfer-(I)-jodid und 7 ml Tetrahydrofuran wurde bei -78°C unter lebhaftem Rühren tropfenweise mit 2,5 ml einer 1,5M Lösung von Methyllithium in Hexan versetzt und dann langsam bis auf 0°C erwärmt. Anschliessend wurde das Gemisch wieder auf -78°C abgekühlt und mittels einer Spritze tropfenweise mit einer 4-Pentenylmagnesiumbromid-Lösung (hergestellt aus 90 mg Magnesium und 0,44 ml 5-Brom-1-penten in 2 ml Tetrahydrofuran) versetzt. Das Reaktionsgemisch wurde allmählich bis zur Bildung einer rotvioletten Suspension aufgewärmt und dann 20 Minuten bei 5°C gehalten. Danach wurde das Reaktionsgemisch wieder auf -78°C abgekühlt, tropfenweise mit einer Lösung von 600 mg trans-4-(p-Cyanophenyl)-jodomethylcyclohexan in 5 ml Tetrahydrofuran versetzt und dann langsam auf Raumtemperatur erwärmt. Das Reaktionsgemisch wurde noch 30 Minuten bei Raumtemperatur gerührt und dann mit gesättigter Ammoniumchlorid-Lösung versetzt und mit Diäthyläther extrahiert. Die organischen Phasen wurden über Magnesiumsulfat getrocknet und eingeengt. Chromatographische Trennung an Kieselgel mit Aethylacetat/Petroläther (Vol. 5:95) und zweimalige Umkristallisation aus Methanol ergab 165 mg p-[trans-4-(5-Hexenyl)cyclohexyl]benzonitril (Reinheit 99,75%) als farblose Kristalle mit Smp. (C-N) 45,5°C und Klp. (N-I) 52,5°C.

In analoger Weise können folgende Verbindungen hergestellt werden:

p-[trans-4-(6-Heptenyl)cyclohexyl]benzonitril; Smp. (C-N) 19,2°C, Klp. (N-I) 32,3°C,
p-[trans-4-(7-Octenyl)cyclohexyl]benzontril; Smp. (C-N) 38,0°C, Klp. (N-I) 53,2°C,
p-[trans-4-(8-Nonenyl)cyclohexyl]benzonitril,
p-[trans-4-(5E- oder 5Z-Heptenyl)cyclohexyl]benzonitril,
p-[trans-4-(5E- oder 5Z-Octenyl)cyclohexyl]benzonitril,
p-[trans-4-(6E- oder 6Z-Octenyl)cyclohexyl]benzonitril,
o-Fluor-p-[trans-4-(5-hexenyl)cyclohexyl]benzonitril,
o-Fluor-p-[trans-4-(6-heptenyl)cyclohexyl]benzonitril,
p-[2-trans-4-(5-Hexenyl)cyclohexyl)äthyl]benzonitril,
p-[2-(trans-4-(6-Heptenyl)cyclohexyl)äthyl]benzonitril,
p-[2-(trans-4-(7-Octenyl)cyclohexyl)äthyl]benzonitril,
p-[2-(trans-4-(5E- oder 5Z-Heptenyl)cyclohexyl)äthyl]benzonitril,
o-Fluor-p-[2-(trans-4-(5-hexenyl)cyclohexyl)äthyl]benzonitril.

Beispiel 2

a) In einem Rundkolben mit Magnetrührer und Rückflusskühler wurde unter Argonbegasung ein Gemisch von 2,61 g p-[trans-4-(5-Hexenyl)cyclohexyl]benzonitril und 20 g Kaliumhydroxid in 200 ml Diäthylenglykol 1 Stunde bei 180°C gekocht. Nach Abkühlen wurde das braune Reaktionsgemisch mit 35 ml 36-prozentiger Salzsäure sauer gestellt und zwischen 100 ml Methylenchlorid und 100 ml Wasser verteilt. Die wässrige Phase wurde dreimal mit je 100 ml Methylenchlorid nachextrahiert.
Die organischen Phasen wurden zweimal mit je 100 ml Wasser gewaschen und über Magnesiumsulfat und Aktivkohle getrocknet. Nach Abtrennung des Lösungsmittels im Rotationsverdampfer wurden 2,93 (104 %))p-[trans-4-(5-Hexenyl)cyclohexyl]benzoesäure als bräunliche, nicht ganz reine Kristalle erhalten; Rf-Wert 0,38 (Aethylacetat/Petroläther Vol. 3:7).

b) Eine Suspension von 2,8 g p-[trans-4-(5-Hexenyl)cyclohexyl] benzoesäure in 50 ml Aceton und 7 ml Wasser wurde auf 0°C gekühlt und langsam mit einer Lösung von 2,73 ml Triäthylamin in 15 ml Aceton versetzt, wobei sich eine klare Lösung bildete. Anschliessend wurde das Gemisch tropfenweise mit einer Lösung von 2,33 ml Chlorameisensäureäthylester in 10 ml Acetone versetzt, wobei ein weisser flockiger Niederschlag ausfiel. Das Gemisch wurde noch 30 Minuten bei 0°C gerührt, dann tropfenweise mit einer Lösung von 1,74 g Natriumazid in 7 ml Wasser versetzt und noch 1 Stunde bei 0°C weitergerührt. Danach wurde das Reaktionsgemisch zwischen 150 ml Methylenchlorid und 150 ml Wasser verteilt. Die wässrige Phase wurde dreimal mit je 150 ml Diäthyläther nachextrahiert. Die organischen Phasen wurden zweimal mit je 100 ml Wasser gewaschen und über Magnesiumsulfat getrocknet. Nach Abtrennen des Lösungsmittels am Rotationsverdampfer wurden 3,1 g (100%) p-[trans-4-(5-Hexenyl)cyclohexyl]benzoesäureazid als feste kristalline Masse erhalten, welche roh weiterverwendet wurde; Rf-Wert 0,63 (Aethylacetat/Petroläther Vol. 1:9).

c) Ein Gemisch von 3,1 g rohem p-[trans-4-(5-Hexenyl)cyclohexylbenzoesäureazid und 100 ml Propanol wurde unter Argonbegasung 1 Stunde am Rückfluss gekocht und danach eingedampft. Hierbei wurden 3,33 g (97%) Propyl-p-[trans-4-(5-Hexenyl)cyclohexyl]phenylcarbamat als hellbraune, feste Masse erhalten, welche roh weiterverwendet wurde; Rf-Wert 0,39 (Aethylacetat/Petroläther Vol. 1:9).

d) Ein Gemisch von 3,33 g rohem Propyl-p-[trans-4-(5-Hexenyl)cyclohexyl]phenylcarbamat und 150 ml einer 10-prozentigen Lösung von Kaliumhydroxid in Diäthylenglykol/Wasser (Vol. 4:1) wurde 2 Stunden bei 120°C gekocht, dann auf Raumtemperatur abgekühlt und zwischen 150 ml Methylenchlorid und 150 ml Wasser verteilt. Die wässrige Phase wird zweimal mit je 150 ml Methylenchlorid nachextrahiert. Die organischen Phasen wurden zweimal mit je 100 ml Wasser gewaschen, über Magnesiumsulfat getrocknet und eingeengt. Neiderdruckchromatographie (0,5 bar) des braunen, kristallinen Rückstandes (3 g) an 70 g Kieselgel mit Aethylacetat/Petroläther (Vol. 20:80) ergab 2,18 g (88%) p-[trans-4-(5-Hexenyl)cyclohexl]anilin als leicht gelbliche Kristalle (Reinheit 98,1%); Rf-Wert 0,20 (Aethylacetat/Petroläther Vol. 1:5).

e) Eine Lösung von 2,36 ml Triäthylamin in 15 ml Chloroform wurde bei -10°C tropfenweise mit einer Lösung von 2,18 g p-[trans-4-(5-Hexenyl)cyclohexyl]anilin in 20 ml Chloroform versetzt. Nach 5 Minuten wurde das Gemisch mit einer Lösung von 690 µl Thiophosgen in 10 ml Chloroform versetzt, auf Raumtemperatur erwärmt und 2 Stunden gerührt. Danach wurde das Reaktionsgemisch zwischen Wasser und

Chloroform verteilt. Die organische Phase wurde über Magnesiumsulfat getrocknet und eingeengt. Chromatographische Trennung an 140 g Kieselgel und Aethylacetat/Petroläther (Vol. 5:95) und anschliessende Kristallisation aus 80 ml Methanol und Tieftemperaturfiltration ergab 1,24 g p-[trans-4-(5-Hexenyl)cyclohexyl]phenylisothiocyanat (Reinheit 100%) als milchige Flüssigkeit mit Smp. (C-N) 15,2°C und Klp. (N-I) 46,1°C.

p-[trans-4-(5-Hexenyl)cyclohexyl]phenylisothiocyanat besitzt bei 22°C eine Bulkviskosität $\eta$ von 12,1 cP, eine optische Anisotropie $\Delta n$ von 0,180 und eine Ausschaltzeit $t_{off}$ von 26ms in einer Drehzelle mit 8 $\mu$m Elektrodenabstand. Sie ist somit weniger viskos und ermöglicht wesentlich kürzere Schaltzeiten als das entsprechende Hexylderivat. Die entsprechenden Werte für p-(trans-4-Hexylcyclo hexyl)phenylisothiocyanat sind: $\eta$ = 16,0 cP, $\Delta$ = 0,153, $t_{off}$ = 41ms.

In analoger Weise können folgende Verbindungen hergestellt werden:

p-[trans-4-(6-Heptenyl)cyclohexyl]phenylisothiocyanat;
Smp. (C-N)1,6°C, Klp. (N-I) 19,0°C,
p-[trans-4-(7-Octenyl)cyclohexyl]phenylisothiocyanat;
Smp. (C-N) 24,6°C, Klp. (N-I) 45,5°C,
p-[trans-4-(8-Nonenyl)cyclohexyl]phenylisothiocyanat,
p-[trans-4-(5E- oder 5Z-Heptenyl)cyclohexyl]phenylisothiocyanat,
p-[trans-4-(5E- oder 5Z-Octenyl)cyclohexyl]phenylisothiocyanat,
p-[trans-4-(6E- oder 6Z-Octenyl)cyclohexyl]phenylisothiocyanat,
o-Fluor-p-[trans-4-(5-hexenyl)cyclohexyl]phenylisothiocyanat,
o-Fluor-p-[trans-4-(6-heptenyl)-cyclohexyl]phenylisothiocyanat,
p-[2-(trans-4-(5-Hexenyl)cyclohexyl)äthyl]phenylisothiocyanat,
p-[2-(trans-4-(6-Heptenyl)cyclohexyl)äthyl]phenylisothiocyanat,
p-[2-(trans-4-(7-Octenyl)cyclohexyl)äthyl]phenylisothiocyanat,
p-[2-(trans-4-(5E- oder 5Z-Heptenyl)cyclohexyl)äthyl]phenylisothiocyanat,
p-Fluor-p-[2-(trans-4-(5-hexenyl)cyclohexyl)äthyl]phenylsiothiocyanat,
p-[5-(5-Hexenyl)-2-pyrimidinyl]phenylisothiocyanat,
p-[5-(6-Heptenyl)-2-pyrimidinyl]phenylisothiocyanat,
p-[5-(7-Octenyl)-2-pyrimidinyl]phenylisothiocyanat,
p-[5-(8-Nonenyl)-2-pyrimidinyl]phenylisothiocyanat,
p-[5-(5E- oder 5Z-Heptenyl)-2-pyrimidinyl]phenylisothiocyanat,
p-[5-(5E- oder 5Z-Octenyl)-2-pyrimidinyl]phenylisothiocyanat,
p-Fluor-p-[5-(5-hexenyl)-2-pyrimidinyl]phenylisothiocyanat.

Beispiel 3

a) Eine Suspension von 22,3 g Methoxymethyl-triphenylphosphoniumchlorid in 60 ml Diäthyläther wird unter Rühren mit 7,62 g Kalium-t-butylat versetzt. 45 Minuten später wird das Gemisch mit einer Lösung von 3,16 g 6-Heptenal in 30 ml Diäthyläther versetzt. Nach 2 Stunden bei Raumtemperatur wird das Reaktionsgemisch auf Eiswasser gegossen. Die organische Phase wird abgetrennt, mit Wasser gewaschen und getrocknet und der erhaltene rohe 1,7-Octadienyl-methyläther destilliert.

b) Eine Lösung von 1,31 g Bortrifluorid-Aetherat in 50 ml destilliertem Orthoameisensäuremethylester wird unter Kühlen mit einem Eisbad tropfenweise mit 2,58 g 1,7-Octadienylmethyläther versetzt. Das Reaktionsgemisch wird über Nacht bei Raumtemperatur gerührt und dann mit 100 ml Toluol verdünnt, mit Natriumhydrogencarbonat-Lösung und Wasser gewaschen und getrocknet. Nach Abdampfen des Lösungsmittels wird rohes (5-Hexenyl)malon-tetraacetal erhalten.

c) Ein Gemisch von 3,77 g rohem (5-Hexenyl)malon-tetraacetat, 0,35 ml Wasser und 15 mg p-Toluolsulfonsäure wird unter Rühren 3 Stunden auf 80-85°C erhitzt. Nach Abkühlung wird das Gemisch mit 160 mg Natriumhydrogencarbonat versetzt und 1,5 Stunden gerührt. Anschliessend wird das Reaktionsgemisch mit Diäthyläther verdünnt, dreimal mit kalter 3N Natronlauge extrahiert, mit Wasser gewaschen und getrocknet. Das nach Abdampfen des Lösungsmittels erhaltene rohe 3-Methoxy-2-(5-hexenyl)acrolein wird ohne weitere Reinigung weiterverarbeitet.

d) Eine Lösung von 480 mg Natrium in 17,5 ml Methanol wird mit 1,18 g 3-Methoxy-2-(5-hexenyl)acrolein und 1,62 g 4-Amidinobenzamid-Hydrochlorid versetzt und unter Stickstoff bei 50°C über Nacht gerührt. Nach Abkühlung wird die Suspension mit 5,5 ml 3N Salzsäure versetzt, filtriert und der Rückstand mit Wasser gewaschen und getrocknet. Das erhaltene rohe p-[5-(5-Hexenyl)-2-pyrimidinyl]benzamid wird ohne zusätzliche Reinigung weiterverwendet.

e) Eine Suspension von 1,37 rohem p-[5-(5-Hexenyl)-2-pyrimidinyl]benzamid in 15 ml Pyridin wird mit 0,95 ml Benzolsulfochlorid versetzt und 6 Stunden auf 55°C erwärmt. Anschliessend wird das Reaktionsgemisch auf kalte verdünnte Salzsäure gegossen und mit Methylenchlorid extrahiert. Die organische Phase wird nacheinander mit Salzsäure, Natriumhydrogencarbonat-Lösung und Wasser gewaschen und dann getrocknet. Nach Abdampfen des Lösungsmittels wird das rohe p-[5-(5-Hexenyl)-2-pyrimidinyl]benzonitril an Kieselgel mit Toluol/Aceton chromatographisch gereinigt und umkristallisiert.

Gewünschtenfalls kann das p-[5-(5-Hexenyl)-2-pyrimidinyl]benzamid in analoger Weise zu Beispiel 2a direkt zur p-[5-(5-Hexenyl)-2-pyrimidinyl]benzoesäure hydrolysiert werden.

In analoger Weise können folgende Verbindungen hergestellt werden:

p-[5-(6-Heptenyl)-2-pyrimidinyl]benzonitril,
p-[5-(7-Octenyl)-2-pyrimidinyl]benzonitril,
p-[5-(8-Nonenyl)-2-pyrimidinyl]benzonitril,
p-[5-(5E- oder 5Z-Heptenyl)-2-pyrimidinyl]benzonitril,
p-[5-(5E- oder 5Z-Octenyl)-2-pyrimidinyl]benzonitril.
o-Fluor-p-[5-(5-hexenyl)-2-pyrimidinyl]benzonitril.

**Patentansprüche**

1. Verbindungen der allgemeinen Formel

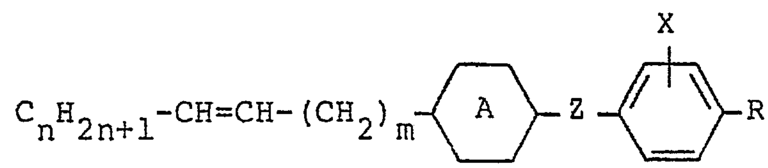

IA

worin m für eine ganze Zahl von 4-7 und n für null oder eine positive ganze Zahl steht; Ring A trans-1,4-Cyclohexylen oder einen 2,5-disubstituierten Pyrimidinring darstellt; Z eine einfache Kovalenzbindung oder, sofern Ring A trans-1,4-Cyclohexylen darstellt, auch die Aethylengruppe $-CH_2CH_2-$ bedeutet; X Wasserstoff oder Fluor bezeichnet; und R die Gruppe -NCS oder -CN darstellt.

2. Verbindungen nach Anspruch 1, dadurch gekennzeichnet, dass R die Gruppe -NCS darstellt.

3. Verbindungen nach Anspruch 1 oder 2, dadurch gekennzeichnet, dass Z eine einfache Kovalenzbindung bedeutet.

4. Verbindungen nach einem der Ansprüche 1 bis 3, dadurch gekennzeichnet, dass Ring A trans-1,4-Cyclohexylen darstellt.

5. Verbindungen nach einem der Ansprüche 1 bis 4, gekennzeichnet durch die allgemeine Formel

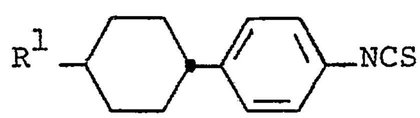

Ia

worin $R^1$ für $C_nH_{2n+1}-CH=CH-(CH_2)_m-$ steht und m und n die in Anspruch 1 gegebenen Bedeutungen haben.

6. Verbindungen nach einem der Ansprüche 1 bis 5, dadurch gekennzeichnet, dass die Summe m+n höchstens 10 beträgt.

7. Verbindungen nach einem der Ansprüche 1 bis 6, dadurch gekennzeichnet, dass der Rest $C_nH_{2n+1}$ Wasserstoff oder geradkettiges Alkyl bedeutet.

8. Verbindungen nach einem der Ansprüche 1 bis 7, dadurch gekennzeichnet, dass m für die Zahl 4 steht.

9. Flüssigkristallines Gemisch mit mindestens 2 Komponenten, dadurch gekennzeichnet, dass mindestens eine Komponente eine Verbindung der in Anspruch 1 definierten Formel IA ist.

10. Verwendung der Verbindungen der in Anspruch 1 definierten Formel IA für elektro-optische Zwecke.

**Claims**

1. Compounds of the general formula

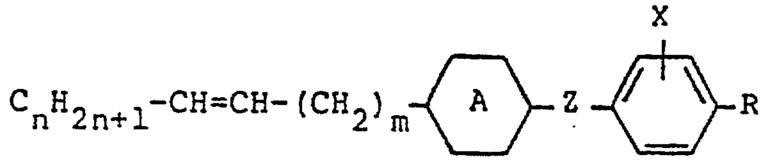

IA

wherein m stands for a whole number of 4–7 and n stands for zero or a positive whole number; ring A represents trans-1,4-cyclohexylene or a 2,5-disubstituted pyrimidine ring; Z signifies a single covalent

bond or, insofar as ring A represents trans-1,4-cyclohexylene, also the ethylene group $-CH_2CH_2-$; X denotes hydrogen or fluorine; and R represents the group –NCS or –CN.

2. Compounds according to claim 1, characterized in that R represents the group –NCS.

3. Compounds according to claim 1 or 2, characterized in that Z signifies a single covalent bond.

4. Compounds according to any one of claims 1 to 3, characterized in that ring A represents trans-1,4-cyclohexylene.

5. Compounds according to any one of claims 1 to 4, characterized by the general formula

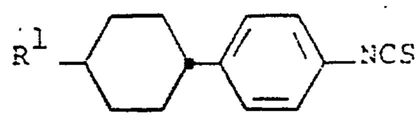

Ia

wherein $R_1$ stands for $C_nH_{2n+1}-CH=CH-(CH_2)_m$ and m and n have the significances given in claim 1.

6. Compounds according to any one of claims 1 to 5, characterized in that the sum m + n amounts to a maximum of 10.

7. Compounds according to any one of claims 1 to 6, characterized in that the residue $C_nH_{2n+1}$ signifies hydrogen or straight-chain alkyl.

8. Compounds according to any one of claims 1 to 7, characterized in that m stands for the number 4.

9. A liquid crystalline mixture with at least 2 components, characterized in that at least one component is a compound of formula IA defined in claim 1.

10. The use of the compounds of formula IA defined in claim 1 for electro-optical purposes.

**Revendications**

1. Composés de formule générale

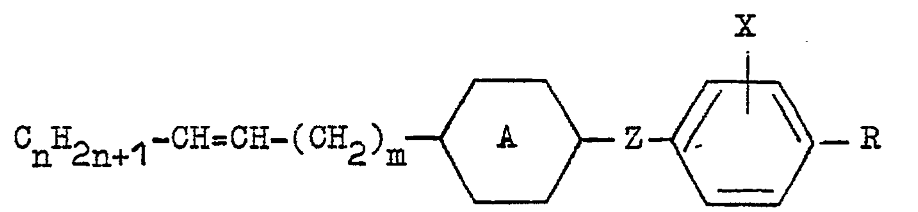

IA

dans laquelle m représente un nombre entier de 4 à 7 et n est égal à 0 ou à un nombre entier positif; le cycle A est un cycle trans-1,4-cyclohexylène ou un cycle pyrimidine 2,5-disubstitué; Z représente une liaison covalente simple ou bien encore, lorsque le cycle A est un cycle trans-1,4-cyclohexylène, le groupe éthylène $-CH_2CH_2-$; X représente l'hydrogène ou le fluor; et R représente un groupe –NCS ou –CN.

2. Composés selon la revendication 1, caractérisés en ce que R représente le groupe –NCS.

3. Composés selon la revendication 1 ou 2, caractérisés en ce que Z représente une liaison covalente simple.

4. Composés selon l'une des revendications 1 à 3, caractérisés en ce que le cycle A est un cycle trans-1,4-cyclohexylène.

5. Composés selon l'une des revendications 1 à 4, caractérisés par la formule générale

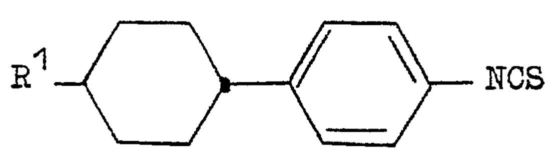

Ia

dans laquelle $R_1$ représente un groupe $C_nH_{2n+1}-CH=CH-(CH_2)_m-$ et m et n ont les significations indiquées dans la revendication 1.

6. Composés selon l'une des revendications 1 à 5, caractérisés en ce que la somme m + n est égale à 10 au maximum.

7. Composés selon l'une des revendications 1 à 6, caractérisés en ce que $C_nH_{2n+1}$ représente l'hydrogène ou un groupe alkyle à chaîne droite.

8. Composés selon l'une des revendications 1 à 7, caractérisés en ce que m est égal à 4.

9. Mélange à cristaux liquides à au moins deux composants, caractérisé en ce que l'un au moins des composants est un composé de formule IA définie dans la revendication 1.

10. Utilisation des composés de formule IA définie dans la revendication 1 dans des applications électro-optiques.